# EUROPÄISCHE PATENTSCHRIFT

(11) **EP 3 405 150 B1**
(45) Veröffentlichungstag und Bekanntmachung des Hinweises auf die Patenterteilung: **23.10.2019**
(21) Anmeldenummer: 17701031.1
(22) Anmeldetag: 20.01.2017
(51) Int. Cl.: A61F 13/00, A61M 1/00, B32B 1/08, B32B 7/00, B32B 37/00

(54) **VERFAHREN ZUM HERSTELLEN EINES FOLIENSCHLAUCHS**
METHOD FOR PRODUCING A TUBULAR FILM
PROCÉDÉ DE FABRICATION D'UN FILM TUBULAIRE

(30) Priorität: 20.01.2016 DE 102016000569
(43) Veröffentlichungstag der Anmeldung: 28.11.2018
(73) Patentinhaber: Lohmann & Rauscher GmbH, 2525 Schönau/Triesting (AT)
(72) Erfinder: STEINLECHNER, Erik, 2500 Baden (AT)
(74) Vertreter: Seranski, Klaus
(86) Internationale Anmeldenummer: PCT/EP2017/000076
(87) Internationale Veröffentlichungsnummer: WO 2017/125250

(56) Entgegenhaltungen:
- EP-A2- 0 807 515
- DE-A1- 10 123 632
- US-A- 4 287 011
- US-A1- 2003 114 782
- US-A1- 2008 132 819
- US-A1- 2010 268 198

## Beschreibung

Die Erfindung betrifft ein Verfahren zum Herstellen eines zumindest abschnittsweise wasserdampfdurchlässigen Folienschlauchs für die Wundversorgung mit Hilfe der Unterdrucktherapie.

Derartige Folienschläuche werden beispielsweise zur Herstellung von Wundversorgungsanordnungen der in der EP 2 636 417 beschriebenen Art eingesetzt. Die bekannten Wundversorgungsanordnungen weisen neben dem Folienschlauch auch einen an dem Folienschlauch angebrachten Sauganschluss auf, über den ein Unterdruck im Wundraum erzeugt werden kann.

Es hat sich gezeigt, daß im Besonderen die Heilung chronischer Wunden durch Anlegen von Unterdruck an diese Wunden gefördert werden kann. Dabei hat es sich als vorteilhaft erwiesen, wenn die Wunde mit einem offenporigen Schaum oder Gaze als Füllmaterial abgedeckt bzw. gefüllt wird, die Wunde zur Erzeugung eines die Wunde und ggf. das Füllmaterial enthaltenden abgeschlossenen Wundraums abgedeckt und auf der der Wunde bzw. dem Füllmaterial abgewandten Seite der Abdeckeinrichtung ein Sauganschluss angebracht wird, über den der Wundraum mit einer zum Erzeugen von Unterdruck ausgelegten Saugeinrichtung verbunden werden kann.

Gemäß der EP 2 636 417 wird die Abdeckeinrichtung mit besonderem Vorteil als Folienschlauch verwirklicht, um so eine einfache Anlage der Wundversorgungsanordnung an eine Extremität zu ermöglichen. Ein entsprechender Folienschlauch kann über die Extremität gezogen und bezüglich der Wunde so positioniert werden, daß die Wunde mit der Abdeckeinrichtung dichtend abgedeckt wird. Anschließend kann die Abdeckeinrichtung mit einer Haftfolie an der der Wunde benachbarten Haut befestigt werden. Dazu kann die Haftfolie von der Stützfolie abgezogen werden und derart um ein Ende des Schlauchs gewickelt werden, daß sie einerseits an dem Schlauch und andererseits an der Haut haftet.

Verfahren nach dem Oberbetriff des Patentanspruchs 1 sind in der US 2008/0132819 A1 und der US 2003/0114782 A1 beschrieben.

Entsprechende Folienschläuche können beispielsweise als Blasfolie unter Einsatz einer Ringdüse hergestellt werden. Allerdings hat es sich als problematisch erwiesen, mit diesen Herstellungsverfahren Folienschläuche herzustellen, die an die anatomischen Verhältnisse der Extremitäten angepasst sind, insbesondere etwa kegelstumpfmantelförmig ausgeführt sind.

Angesichts dieser Probleme im Stand der Technik liegt der Erfindung die Aufgabe zugrunde, Verfahren zum Herstellen von als Abdeckeinrichtung für Wundversorgungsanordnungen gemäß EP 2 636 417 einsetzbaren Folienschläuchen bereitzustellen, mit denen an die anatomischen Verhältnisse im Bereich der Wunde angepasste Folienschlauchformen einfach bereitgestellt werden können.

Erfindungsgemäß wird diese Aufgabe durch die im kennzeichnenden Teil des Patentanspruchs 1 angegebene Weiterbildung der bekannten Verfahren gelöst.

Die Erfindung geht auf die Erkenntnis zurück, daß die Anforderungen an einen Folienschlauch für die Wundversorgung hinsichtlich der Wasserdampfdurchlässigkeit zum einen und der Luft- und Flüssigkeitsundurchlässigkeit zum anderen auch durch Nähte aufweisende Folienschläuche erfüllt werden können, wenn der Schlauch nicht bei der Herstellung der Folie selbst, sondern durch eine Weiterverarbeitung einer ebenen Folie erhalten wird.

Bei erfindungsgemäßen Verfahren kann die Schlauchform, wie etwa eine kegelstumpfmantelförmige Schlauchform, durch passende Wahl der Faltlinien und Verbindungslinien bestimmt werden. Dadurch wird eine Anpassung an die anatomischen Verhältnisse im Bereich der Wunde möglich. Andererseits wird das Herstellungsverfahren durch Einsatz der Stützfolie erleichtert, welche die gewünschte Formgebung auch bei Einsatz von wasserdampfdurchlässigen Folien mit einer Dicke von nur 0,5 bis 200 µm, insbesondere 1 bis 100 µm ermöglichen. Dem Laminat wird mit Hilfe der Stützfolie auch bei Einsatz entsprechend dünner wasserdampfdurchlässiger Folien, wie etwa PU-Folien, die notwendige Stabilität verliehen, welche eine Faltung bzw. eine Verschweißung längs vorgegebener Falt- bzw. Verbindungslinien ermöglicht. Im Rahmen erfindungsgemäßer Verfahren können Laminate eingesetzt werden, bei denen Stützfolien aus Polyester vorgesehen sind. Diese Stützfolien können beidseitig mit PU beschichtet und ggf. einseitig silikonisiert sein. Die wasserdampfdurchlässige Folie, wie etwa die PU-Folie, kann auf der silikonisierten Seite der Stützfolie vorgesehen sein und durch Adhäsion an der Stützfolie haften bzw. befestigt sein.

Zweckmäßigerweise wird das Material der Stützfolie so gewählt, daß es bei höheren Temperaturen aufschmilzt als das Material der wasserdampfdurchlässigen Folie. Die vorstehend beispielhaft angegebene Polyesterfolie kann eine Zersetzungstemperatur von 350° bis 400° C aufweisen, während die vorstehend als Beispiel für eine wasserdampfdurchlässige Folie genannten PU-Folie oder auch PUR-Folie eine Zersetzungstemperatur von 200° bis 220° C aufweisen kann. Dann können die wasserdampfdurchlässigen Folien selektiv miteinander verbunden werden, ohne daß es zu einem Aufschmelzen der Stützfolien selbst kommt. Diese Materialwahl ermöglicht die Entfernung der Stützfolienlagen von den miteinander verbundenen wasserdampfdurchlässigen Folienlagen, ohne Beschädigung der Verbindung zwischen den wasserdampfdurchlässigen Lagen, wobei zweckmäßigerweise ein auf der der Faltlinie abgewandten Seite der Verbindungslinien gebildeter Randstreifen der wasserdampfdurchlässigen Folien zusammen mit den Schutzfolienlagen von dem Schlauch entfernt wird.

Diese Entfernung des Randstreifens wird bei Einsatz einer Ultraschallschweißanlage zum Verbinden der wasserdampfdurchlässigen Folienlagen erfindungsgemäß begünstigt, weil das aufgeschmolzene Material der wasserdampfdurchlässigen Lage durch die Temperaturführung und/oder die Form der Sonotroden der Ultraschallschweißanlage während des Schweißvorgangs überwiegend in Richtung der Faltlinie fließt, wo es dann eine entsprechend stabile Verbindung zwischen den wasserdampfdurchlässigen Folienlagen bildet.

Dieses günstige Fließverhalten des aufgeschmolzenen, wasserdampfdurchlässigen Materials kann besonders einfach erreicht werden, wenn die Folienlagen zum Herstellen der Verbindung zwischen den wasserdampfdurchlässigen Folien einen vorzugsweise zwischen einer Sonotrodenrolle und einer Ambossrolle gebildeten Schweißspalt einer Ultraschallschweißanlage durchlaufen, wobei die Sonotrodenrolle und/oder die Ambossrolle während des Schweiß- bzw. Verbindungsvorgangs in eine senkrecht zur Verbindungslinie und parallel zu den Folienlagen verlaufende Drehachse gedreht werden. Dann werden die miteinander zu verbindenden Folienlagen durch Drehung der Sonotrodenrolle und/oder Ambossrolle gleichzeitig längs der Verbindungslinie gefördert, während das aufgeschmolzene Folienmaterial quer dazu in Richtung auf die Falllinie strömt und sich zur Herstellung einer stabilen Verbindung verfestigen kann. Das Fließverhalten des aufgeschmolzenen Folienmaterials kann auch durch geeignete Profilierung der Amboss- und/oder Stützrolle beeinflusst werden. Maßgeblich für die Steuerung des Fließverhaltens sind Anpressdruck der beiden Rollen, Vorschub des zu verbindenden Materials und die Amplitude der Ultraschallschwingung.

Auf diese Weise wird an dem der Faltlinie zugewandten Rand des zwischen Sonotrodenrolle und Ambossrolle geförderten Randstreifens der wasserdampfdurchlässigen Folienlagen eine Verbindungslinie zwischen diesen Folienlagen gebildet, längs der diese Folien fester miteinander verbunden sind als im übrigen Randstreifen. Durch diese Struktur wird die Entfernung des Randstreifens der wasserdampfdurchlässigen Folienlagen zusammen mit den Stützfolienlagen begünstigt, wobei nur die Schweißnaht längs der Verbindungslinie als Verbindung zwischen den einzelnen Folienlagen verbleibt.

Zusätzlich oder alternativ zu einer Ultraschallschweißanlage mit Sonotrodenrolle und Ambossrolle können auch Anlagen zum Einsatz kommen, bei denen Sonotrode und Amboss jeweils in einer Ebene, ähnlich wie bei einem Stempel, aufeinander gepresst und die Verschweißung so bewirkt wird. Auch in diesem Fall kann die Form der Schweißnaht durch Profilierung von Sonotrode und/oder Amboss und Anpressdruck von Sonotrode und Amboss gesteuert werden.

Die Schweißnaht kann nach Entfernen der Stützfolie mit Hilfe eines Verstärkungsstreifens gesichert werden, der im Bereich der Verbindungslinie auf die wasserdampfdurchlässige Folie aufgebracht wird. Dazu kann der Folienschlauch derart auf eine Stützanordnung aufgezogen werden, daß die Verbindungslinie längs der die wasserdampfdurchlässigen Folienlagen miteinander verbunden sind, von einer Stützfläche der Stützanordnung abgestützt wird, so daß die wasserdampfdurchlässigen Folien bei Aufbringen des Verstärkungsstreifens auf die Verbindungslinie abgestützt werden und so vor Beschädigungen im Bereich der Verbindungslinie geschützt sind.

Ein derartiger Streifen kann eine mit einem Haftmittel ausgestattete Siegelfolie aufweisen, wobei auf der dem Haftmittel abgewandten Begrenzungsfläche der Siegelfolie ein Stützstreifen vorgesehen sein kann, der nach Aufbringen der Siegelfolie auf die Verbindungslinie von der Siegelfolie entfernt werden kann.

Nachstehend wird die Erfindung unter Bezugnahme auf die Zeichnung, auf die hinsichtlich aller erfindungswesentlicher Einzelheiten, die in der Beschreibung nicht ausdrücklich hervorgehoben sind, Bezug genommen wird, erläutert.

In der Zeichnung zeigt
- Fig. 1: eine Darstellung einer zur Ausführung erfindungsgemäßer Verfahren einsetzbaren Ultraschallschweißmaschine und
- Fig. 2: Darstellungen zur Veranschaulichung der einzelnen Verfahrensschritte erfindungsgemäßer Verfahren.

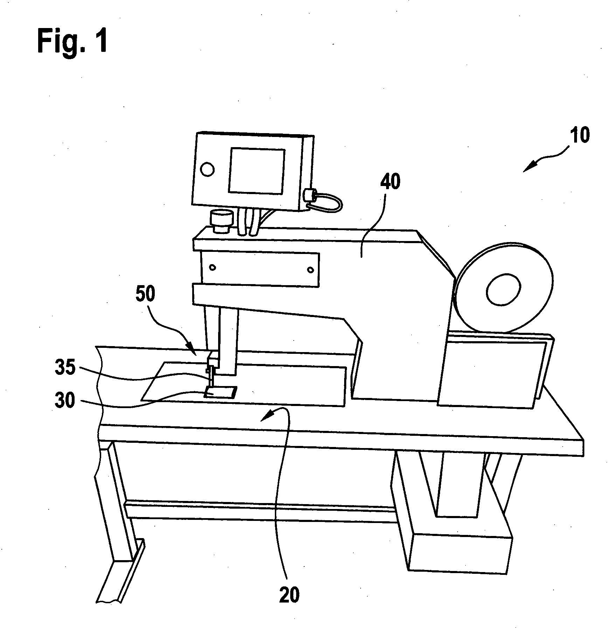

Die in Fig. 1 dargestellte Ultraschallschweißmaschine umfasst einen Arbeitstisch 20, eine Sonotrodenrolle 30, die in einer in dem Arbeitstisch 20 gebildeten Schlitz aufgenommen ist und eine Ambossrolle 35, die bezüglich einer parallel zum Arbeitstisch 20 verlaufenden Drehachse drehbar an einem Ausleger 40 gehalten ist. Zwischen dem Ausleger 40 und dem Arbeitstisch ist ein Arbeitsraum 50 gebildet, durch den die miteinander zu verbindenden Folienlagen geführt werden können.

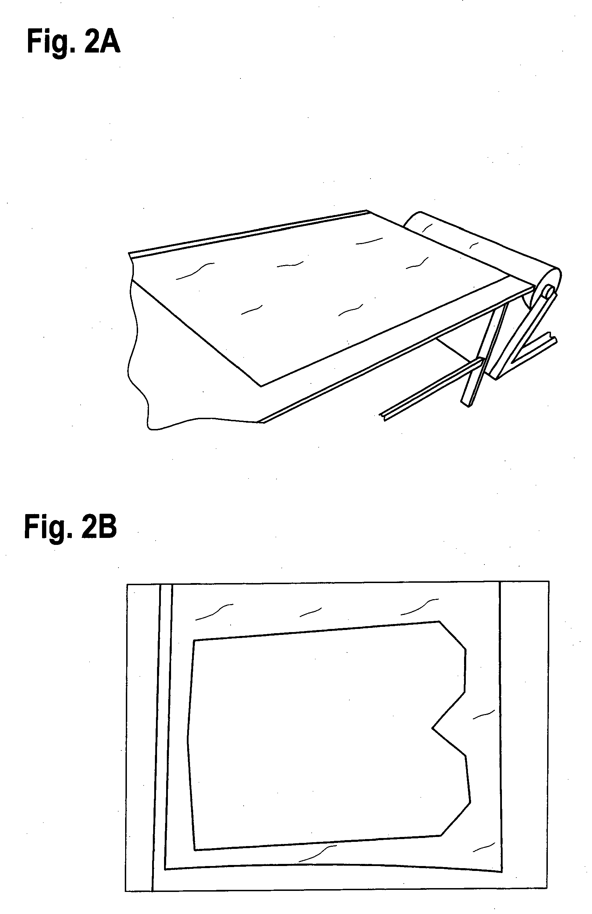

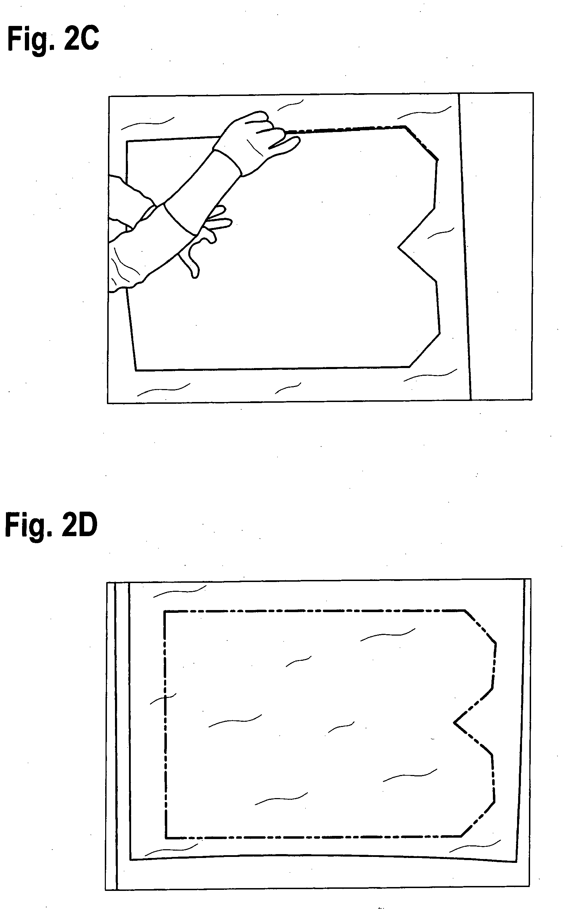

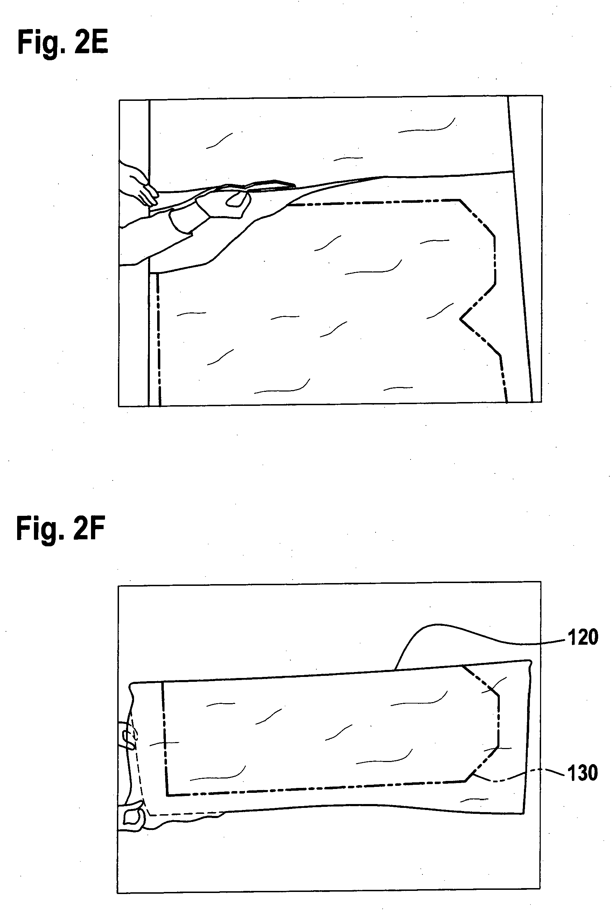

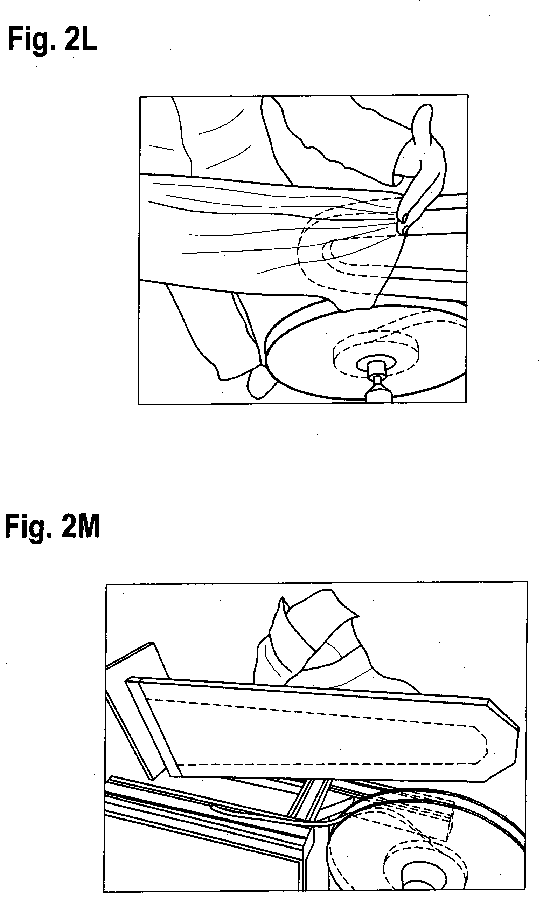

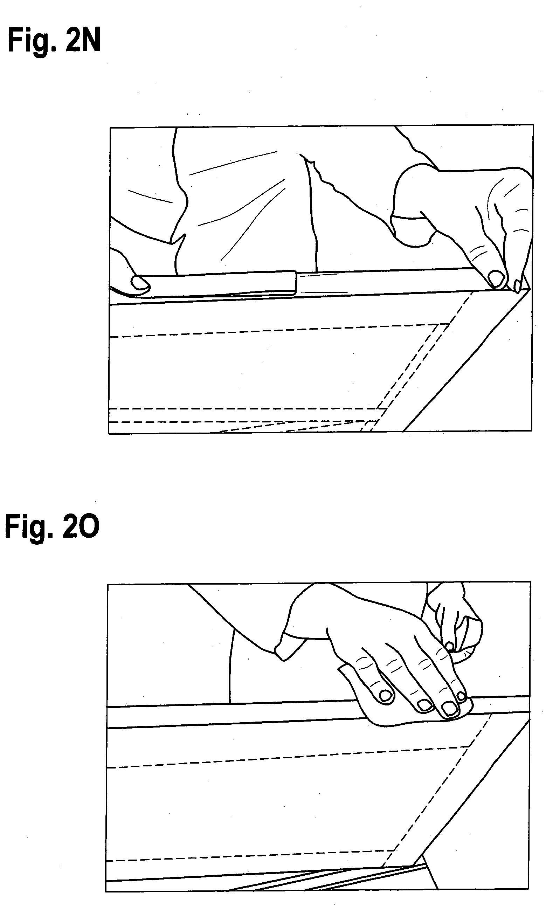

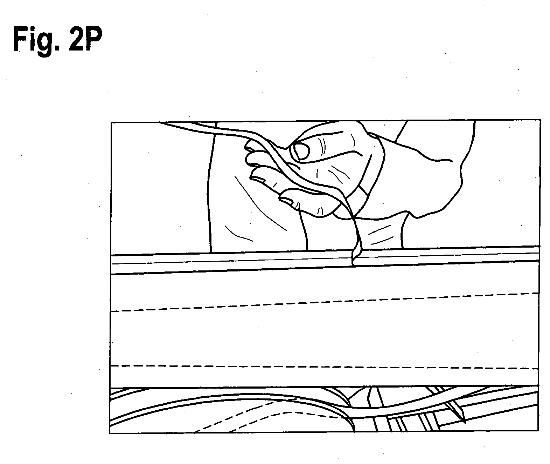

Gemäß Fig. 2A wird in einem ersten Arbeitsschritt bei der Ausführung erfindungsgemäßer Verfahren das als Laminat ausgeführte Ausgangsmaterial auf einem ebenen Zuschneidetisch ausgerollt. Hierbei liegt die Stützfolie zweckmäßigerweise auf dem Zuschneidetisch auf, während die dem Zuschneidetisch abgewandte Begrenzungsfläche des Ausgangsmaterials durch die wasserdampfdurchlässige PU-Folie gebildet wird. Auf das so ausgebreitete Ausgangsmaterial wird eine Schablone 110 aufgelegt und mit Hilfe dieser Schablone ein Zuschnitt des Ausgangsmaterials entsprechend der gewünschten Folienschlauchform angezeichnet, wie in den Fig. 2B und 2C angedeutet.

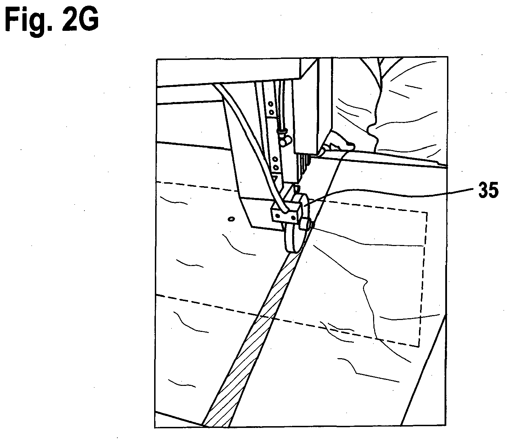

Nach Entfernen der Schablone erfolgt ein grober Zuschnitt des Ausgangsmaterials, wie durch die Fig. 2D und 2E angedeutet. Anschließend wird das Ausgangsmaterial längs einer Faltlinie 120 derart gefaltet, daß zwei wasserdampfdurchlässige Folienlagen zwischen zwei Stützlagen zu liegen kommen und die vorher angezeichneten Schnittlinien 130 übereinanderliegen. Anschließend werden die zwischen den Stützfolienlagen angeordneten Lagen aus wasserdampfdurchlässigen Folien mit Hilfe der Ultraschallschweißmaschine längs der Schnittlinien 130 miteinander verschweißt, wie in Fig. 2G angedeutet. Dazu werden die Folienlagen zwischen der Ambossrolle 35 und einer nicht dargestellten Sonotrodenrolle längs der Verbindungslinie gefördert. So wird ein Randstreifen der wasserdampfdurchlässigen Folienlagen mit einer Breite von etwa einem Zentimeter gebildet, der auf der der Faltlinie 120 zugewandten Seite von einer Verbindungslinie begrenzt wird. Das aufgeschmolzene Material der wasserdampfdurchlässigen Folienlagen fließt in Richtung auf die Faltlinie 120 und sammelt sich im Bereich der Verbindungslinie, wo es sich verfestigt und eine stabile Verbindung zwischen den wasserdampfdurchlässigen Folienlagen bewirkt. Das wird durch die in Fig. 2G dargestellte Profilierung der Umfangsfläche der Ambossrolle begünstigt.

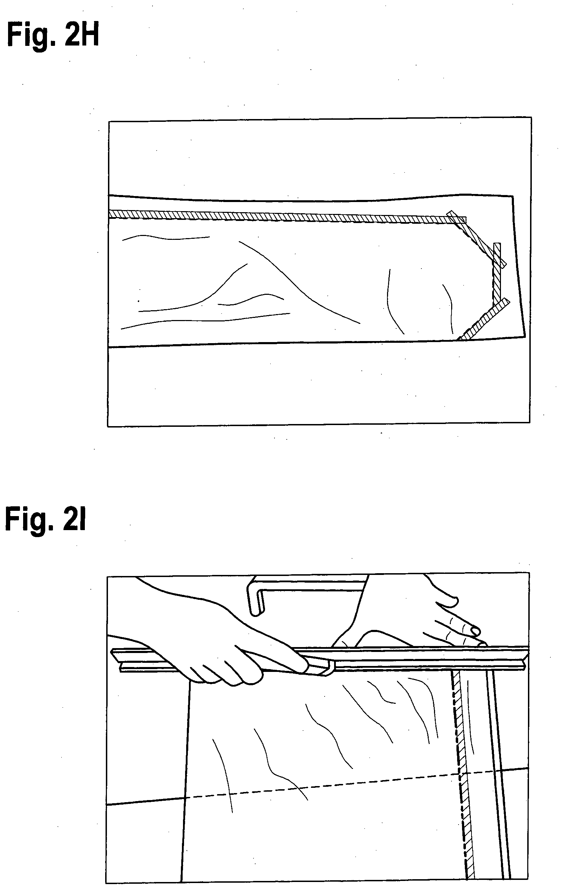

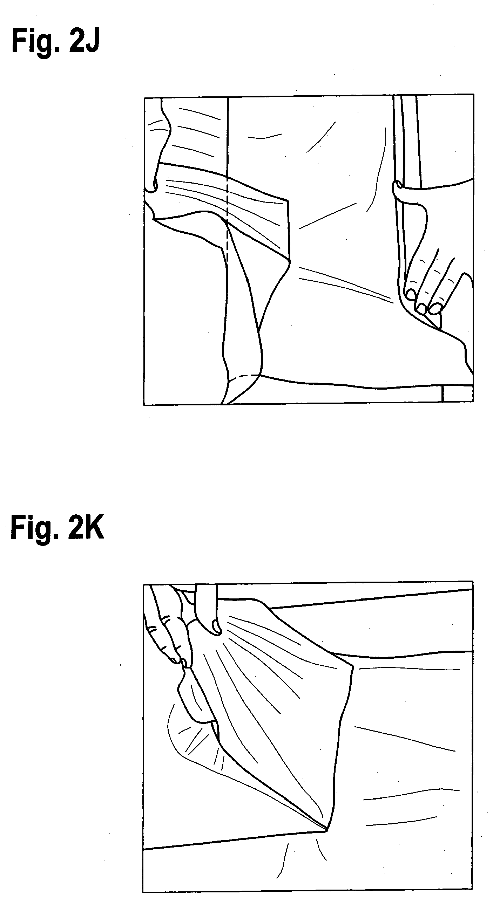

In Fig. 2H ist der Zuschnitt mit verschweißten Verbindungslinien dargestellt. Dieser kann dann auf ein gewünschtes Fertigmaß abgelängt werden, wie in Fig. 2I dargestellt. Anschließend kann die Stützfolie unter Mitnahme des Randstreifens der wasserdampfdurchlässigen Folie von dem Folienschlauch entfernt werden, so daß nur noch ein Folienschlauch aus wasserdampfdurchlässiger Folie verbleibt, der längs der Verbindungslinie geschlossen ist. Dieser Vorgang ist in Fig. 2J und Fig. 2K dargestellt.

Der so erhaltene Folienschlauch wird auf eine bügelförmige Stützeinrichtung aufgezogen, und zwar derart, daß die Verbindungslinie von einer Stützfläche der Stützeinrichtung abgestützt wird. Anschließend wird ein Siegelstreifen über die Verbindungslinie gelegt und dort aufgeklebt, um die Verbindung zu stabilisieren und abzudichten. Endlich wird eine Stützfolie von der Siegelfolie des Siegelstreifens abgezogen, um den Folienschlauch fertigzustellen. Dabei kann der Siegelstreifen mit einem Vliesstoff auf die Verbindungslinie aufgedrückt werden, wobei der Folienschlauch im Bereich der Verbindungslinie von der Stützfläche der Stützeinrichtung abgestützt wird. Das Aufbringen des Siegelstreifens ist in den Fig. 2L bis 2P dargestellt.

Die wasserdampfdurchlässige Folie kann folgende Materialeigenschaften aufweisen:
Die Flächenmasse kann 24 ± 4 g/m² betragen. Die Dicke kann 24 ± 6 µm betragen. Die Wasserdampfdurchlässigkeit kann mehr als 2000 g/m²/24 h betragen. Die Reißkraft kann in Längs- und in Querrichtung 16 ± 6 N/15 mm betragen. Die Reißdehnung kann 700 ± 200 % betragen. Die Trennkraft kann mehr als 30cN/50 mm betragen.

Die Ultraschallschweißanlage kann mit folgenden Betriebsparametern betrieben werden: Die Vorschubgeschwindigkeit des Materials kann 1,9 m/min betragen. Der Anpreßdruck der Schweißsonotrode kann 2,5 bar betragen. Das Vorschubverhältnis von Ambossrolle zu Sonotrodenrolle kann 100 % betragen.

## Patentansprüche

1. Verfahren zum Herstellen eines zumindest abschnittweise wasserdampfdurchlässigen Folienschlauchs für die Wundversorgung mit Hilfe der Unterdrucktherapie, bei dem ein Laminat aus einer Stützfolie und einer wasserdampfdurchlässigen Folie längs einer Faltlinie derart auf sich selbst gefaltet wird, daß zwei wasserdampfdurchlässige Folienlagen zwischen zwei Stützfolienlagen angeordnet sind und die wasserdampfdurchlässigen Folienlagen längs einer Verbindungslinie miteinander verschweißt werden, **dadurch gekennzeichnet, dass** das aufgeschmolzene Material der wasserdampfdurchlässigen Folienlagen durch die Temperaturführung und/oder die Form der Sonotroden der Ultraschallschweißanlagen während des Schweißvorgangs überwiegend in Richtung der Faltlinie fließt, und die Stützfolienlagen von den miteinander verbundenen wasserdampfdurchlässigen Folienlagen entfernt werden, wobei ein auf der der Faltlinie abgewandten Seite der Verbindungslinie angeordneter Randstreifen der wasserdampfdurchlässigen Folien zusammen mit den Stützfolienlagen von dem Folienschlauch entfernt wird.

2. Verfahren nach Anspruch 1, **dadurch gekennzeichnet, daß** die Folienlagen zum Herstellen der Verbindung zwischen den wasserdampfdurchlässigen Folien einen vorzugsweise zwischen einer Sonotrodenrolle und einer Ambossrolle einer Ultraschallschweißanlage gebildeten Schweißspalt durchlaufen.

3. Verfahren nach Anspruch 2, **dadurch gekennzeichnet, daß** die Sonotrodenrolle und/oder die Ambossrolle zum Verbinden der Folienlagen um eine senkrecht zur Verbindungslinie und parallel zu den Folienlagen liegende Drehachse gedreht werden.

4. Verfahren nach einem der vorhergehenden Ansprüche, **dadurch gekennzeichnet, daß** an dem der Faltlinie zugewandten Rand des Randstreifens eine Verbindungslinie zwischen den wasserdampfdurchlässigen Folien gebildet wird, längs der diese Folienlagen fester miteinander verbunden sind als im übrigen Randstreifen.

5. Verfahren nach einem der vorhergehenden Ansprüche, **dadurch gekennzeichnet, daß** nach Entfernen der Stützfolie ein Verstärkungsstreifen im Bereich der Verbindungslinie auf die wasserdampfdurchlässige Folie aufgebracht wird.

6. Verfahren nach einem der vorhergehenden Ansprüche, **dadurch gekennzeichnet, daß** der Folienschlauch zum Aufbringen der Verstärkungsfolie derart auf eine Stützanordnung aufgezogen wird, daß die Verbindungslinie von einer Stützfläche der Stützanordnung abgestützt wird.

## Claims

1. A method for producing a tubular film for wound dressing using negative pressure therapy, with at least some sections of the tubular film being permeable to steam, in which method a laminate consisting of a substrate film and a steam-permeable film is folded onto itself along a fold line in such a way that two steam-permeable film layers are placed between two substrate film layers, and the steam-permeable film layers are welded to each other along a join line, **characterised in that** the molten material of the steam-permeable film layers flows through the temperature control and/or the profile of the sonotrodes of the ultrasonic welding device, predominantly towards the fold line during the welding process, and the substrate film layers are removed from the joined steam-permeable film layers, wherein an edge strip of the steam-permeable films which is located on the side of the join line facing away from the fold line is removed from the tubular film together with the substrate film layers.

2. The method according to claim 1, **characterised in that** in order to produce the join between the steam-permeable films, the film layers pass through a weld gap preferably formed between a sonotrode roller and an anvil roller of an ultrasonic welding device.

3. The method according to claim 2, **characterised in that** in order to join the film layers, the sonotrode roller and/or the anvil roller are rotated around an axis of rotation perpendicular to the join line and parallel to the film layers.

4. The method according to one of the preceding claims, **characterised in that** a join line between the steam-permeable films is formed along the edge of the edge strip facing the fold line, along which join line said film layers are joined to each other more firmly than in the rest of the edge strip.

5. The method according to one of the preceding claims, **characterised in that**, after removing the substrate film, a reinforcement strip is applied to the steam-permeable film in the area of the join line.

6. The method according to one of the preceding claims, **characterised in that** in order to apply the reinforcement film, the tubular film is placed on a support arrangement such that the join line is supported by a supporting surface of the support arrangement.

## Revendications

1. Procédé de fabrication d'un film tubulaire qui, au moins par sections, est perméable à la vapeur d'eau, pour le traitement d'une plaie par pression négative, dans lequel procédé un stratifié constitué d'un film de support et d'un film perméable à la vapeur d'eau est replié sur lui-même le long d'une ligne de pliage de telle manière que deux couches de film perméable à la vapeur d'eau sont disposées entre deux couches de film de support et les couches de film perméable à la vapeur d'eau sont soudées l'une à l'autre le long d'une ligne de liaison, **caractérisé en ce que** le matériau en fusion des couches de film perméable à la vapeur d'eau s'écoule majoritairement en direction de la ligne de pliage, sous l'effet du contrôle de la température et/ou de la forme des sonotrodes des dispositifs de soudage par ultrasons lors du processus de soudage, et les couches de film de support sont retirées des couches de film perméable à la vapeur d'eau liées l'une à l'autre, une bande de bordure des films perméables à la vapeur d'eau, disposée du côté de la ligne de liaison détourné de la ligne de pliage, étant retirée du film tubulaire conjointement aux couches de film de support.

2. Procédé selon la revendication 1, **caractérisé en ce que**, pour produire la liaison entre les films perméables à la vapeur d'eau, les couches de film passent par une fente de soudage formée de préférence entre un rouleau sonotrode et un rouleau enclume d'un dispositif de soudage par ultrasons.

3. Procédé selon la revendication 2, **caractérisé en ce que**, pour relier les couches de film, le rouleau sonotrode et/ou le rouleau enclume subissent une rotation sur un axe de rotation perpendiculaire à la ligne de liaison et parallèle aux couches de film.

4. Procédé selon l'une des revendications précédentes, **caractérisé en ce qu'**une ligne de liaison entre les films perméables à la vapeur d'eau est formée sur le bord de la bande de bordure tourné vers la ligne de pliage, le long de laquelle ligne de liaison ces couches de film sont liées l'une à l'autre plus solidement que dans le reste de la bande de bordure.

5. Procédé selon l'une des revendications précédentes, **caractérisé en ce que**, après le retrait du film de support, une bande de renfort est appliquée dans la zone de la ligne de liaison sur le film perméable à la vapeur d'eau.

6. Procédé selon l'une des revendications précédentes, **caractérisé en ce que**, pour l'application du film de renfort, le film tubulaire est monté sur un dispositif de support de manière que la ligne de liaison soit supportée par une surface de support du dispositif de support.
